(19) Europäisches Patentamt
European Patent Office
Office européen des brevets

(11) Veröffentlichungsnummer: **0 053 687**
**A1**

(12) # EUROPÄISCHE PATENTANMELDUNG

(21) Anmeldenummer: 81108692.5

(22) Anmeldetag: 22.10.81

(51) Int. Cl.³: **C 07 C 17/34**
C 07 C 21/04
//C07C21/073, C07C21/19

(30) Priorität: 10.12.80 DE 3046461

(43) Veröffentlichungstag der Anmeldung: 16.06.82 Patentblatt 82/24

(84) Benannte Vertragsstaaten: BE CH DE FR GB LI

(71) Anmelder: BASF Aktiengesellschaft
Carl-Bosch-Strasse 38
D-6700 Ludwigshafen(DE)

(72) Erfinder: Fischer, Martin, Dr.
Elbingerweg 1
D-6700 Ludwigshafen(DE)

(54) **Verfahren zur Herstellung von 1,1-Dichloralkenylenverbindungen.**

(57) Herstellung von 1,1-Dichloralkenylenverbindungen durch Umsetzung von 1,1,1-Trichlorverbindungen mit wäßrigen Alkalilaugen in Gegenwart von Mono- oder Polyolen.

Die so erhaltenen 1,1-Dichloralkyenylenverbindungen sind Monomere und wertvolle Ausgangsstoffe für die Herstellung von Farbstoffen, Pharmazeutika und Pflanzenschutzmitteln.

EP 0 053 687 A1

Croydon Printing Company Ltd.

Verfahren zur Herstellung von 1,1-Dichloralkenylenverbindungen

Die Erfindung betrifft ein neues Verfahren zur Herstellung von 1,1-Dichloralkenylenverbindungen durch Umsetzung von 1,1,1-Trichlorverbindungen mit wäßrigen Alkalilaugen in Gegenwart von Mono- oder Polyolen.

Die Abspaltung von Chlorwasserstoff aus einfachen Chlorverbindungen mit basischen Mitteln ist bekannt. (Houben-Weyl, Methoden der organischen Chemie, Band 5/4, Seiten 728 - 753). Als basische Mittel werden Alkalihydroxide oder -alkoholate und tert. Amine verwendet. Die Umsetzung mit Hydroxiden führt man ebenfalls in alkoholischen Lösungsmitteln oder in Wasser durch.

Houben-Weyl, Methoden der organischen Chemie, Band 5/4, Seite 731 lehrt, daß man 1,1-Dichloräthylen aus 1,1,2-Trichloräthan mit wäßriger Alkalilauge herstellen kann. Ebenfalls wird eine Synthese durch Zersetzung von 1,1,1-Trichloräthan bei 90 - 130°C mit Eisen-III-chlorid (loc.cit. Seite 735) beschrieben.

Aus wirtschaftlichen Gründen ist die Verwendung von wäßrigen Alkalihydroxiden, d.h. von z.B. Natronlauge oder Kalilauge zu bevorzugen. Leider verläuft die Chlorwasserstoffabspaltung mit wäßrigen Hydroxiden häufig mit schlechter Ausbeute, erfordert sehr lange Reaktionszeiten oder bleibt auch ganz aus.

Houben-Weyl weist ausdrücklich (loc.cit. Seite 744) darauf hin, daß bei Verwendung wäßriger alkalischer Lösungen Solvolyse eintritt. Man verwendet daher zur Dehydrohalogenierung rein alkoholische Lösungen, wobei lediglich als Zusatz zu solchen Lösungen, und zwar zur Zurückdrängung der Ätherbildung, Triäthanolamin, Alkylenglykolmonoalkyl-

äther oder Diäthylenglykol verwendet werden. Besonders wirksam erscheint Houben-Weyl Lösungen der Alkalihydroxide in organischen Lösungsmitteln allein. Die Beispiele zeigen wie spezifisch jedes Lösungsmittel je nach Zahl, Art und Stellung der Halogenatome sowie der Wasserstoffatome und weiterer Substituenten reagiert.

Es wurde nun gefunden, daß man 1,1-Dichloralkenylenverbindungen der Formel I

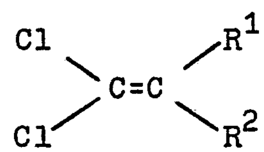 I,

worin $R^1$ und $R^2$ gleich oder verschieden sein können und jeweils ein Wasserstoffatom oder einen aliphatischen Rest bedeuten, $R^1$ auch einen araliphatischen oder aromatischen Rest bezeichnet, durch Umsetzung von 1,1,1-Trichlorverbindungen der Formel II

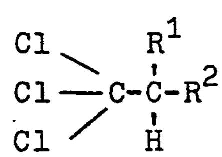 II,

worin $R^1$ und $R^2$ die obengenannte Bedeutung besitzen, mit wäßrigen Alkalilaugen vorteilhaft herstellt, wenn man die Umsetzung in Gegenwart eines Mono- oder Polyols der Formel

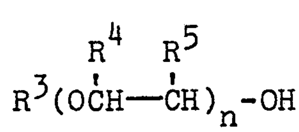 III,

in der $R^3$, $R^4$ und $R^5$ gleich oder verschieden sein können und für ein Wasserstoffatom oder einen aliphatischen Rest stehen und n eine ganze Zahl von 1 bis 10 bedeutet oder der Formel

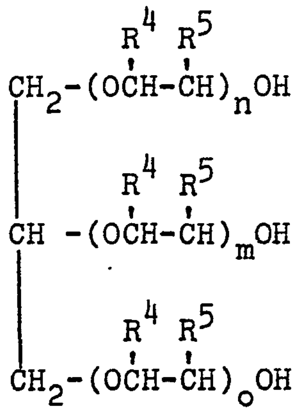

IV,

oder der Formel

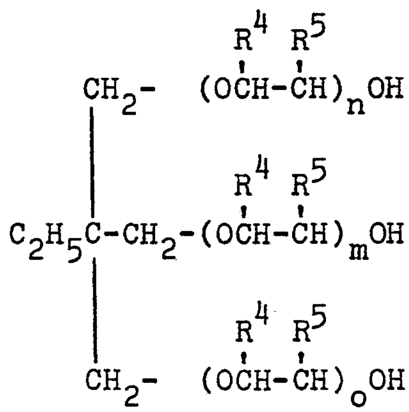

V,

wobei $R^4$, $R^5$ und n die oben angegebene Bedeutung haben und n, m, o gleich oder verschieden sein können und m und o eine ganze Zahl von 0 bis 10 bedeuten, oder der Formel

$$N(CH_2CH_2OH)_3 \qquad VI$$

oder der Formel

$$R^4N(CH_2CH_2OH)_2 \qquad VII,$$

in der $R^4$ die oben angegebene Bedeutung hat, oder der Formel

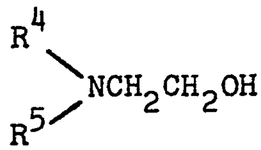

VIII,

in der $R^4$ und $R^5$ die oben angegebene Bedeutung haben, durchführt.

Die Umsetzung wird für den Fall der Verwendung von 1,1,1-trichloräthan durch die folgende Formel wiedergegeben:

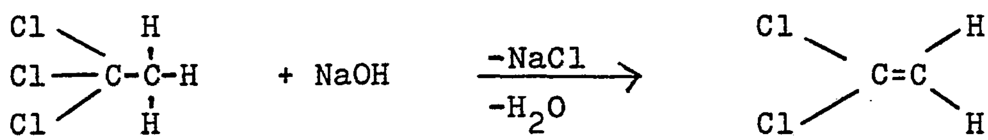


Im Vergleich zu den bekannten Verfahren liefert das Verfahren nach der Erfindung überraschend auf einfacherem und wirtschaftlicherem Wege 1,1-Dichloralkenylene in guter Ausbeute, besserer Raum-Zeitausbeute und Reinheit, gerade auch im großtechnischen Maßstab und im kontinuierlichen Betrieb. Im Hinblick auf den Stand der Technik war es überraschend, daß die bei der Umsetzung mit wäßriger Lauge genannten Schwierigkeiten nicht auftreten und vergleichsweise bessere Ausbeuten und Raum-Zeitausbeuten erhalten werden. Zwar erzielt man in rein methanolischem Alkali ebenfalls gute Ausbeuten, vergleichsweise ist hier das erfindungsgemäße Verfahren einfacher, wirtschaftlicher und wegen Vermeidung von Toxizitäts-, Transport- und Abwasserproblemen wesentlich umweltfreundlicher. Diese vorteilhaften Ergebnisse sind überraschend, denn man hätte bei den erfindungsgemäßen Lösungsmitteln als Zusatz keine Verbesserung der Ergebnisse im wäßrigen Medium erwartet.

Der Ausgangsstoff II kann mit der Lauge in stöchiometrischer Menge oder im Überschuß, vorzugsweise in einem Verhältnis von 1,0 bis 2,0, vorzugsweise 1,2 bis 1,7 Mol Al-

kalihydroxid je Mol Ausgangsstoff II umgesetzt werden. Bevorzugt sind 10 bis 60, vorteilhaft 30 bis 55 gew.%ige wäßrige Alkalilaugen, insbesondere Natron- und Kalilaugen. Bevorzugte Ausgangsstoffe II und dementsprechend bevorzugte Endstoffe I sind solche, in deren Formeln $R^1$ und $R^2$ gleich oder verschieden sein können und jeweils ein Wasserstoffatom, einen gegebenenfalls durch Hydroxygruppen substituierten Alkylrest mit 1 bis 12, insbesondere 1 bis 6 Kohlenstoffatomen, oder Alkenylrest mit 2 bis 12, insbesondere 2 bis 6 Kohlenstoffatomen bezeichnen, $R^1$ auch einen Aralkylrest oder Alkylarylrest mit 7 bis 12 Kohlenstoffatomen oder einen Phenylrest bezeichnet. Die vorgenannten Reste können noch durch unter den Reaktionsbedingungen inerte Gruppen, z.B. Hydroxygruppen, Alkyl- oder Alkoxygruppen mit jeweils 1 bis 4 Kohlenstoffatomen, substituiert sein.

Es kommen beispielsweise folgende Ausgangsstoffe II in Frage: 1,1,1-Trichlor-äthan, -propan, -isopropan, -butan, -isobutan, -sek.-butan, -pentan, -heptan, -hexan, -octan, -nonan, -pentene, -hexene, -heptene, -butene, -decan, -undecan, -dodecan; homologe in $\alpha$-Stellung zur Trichlormethylgruppe durch Methyl-, Äthyl-, Butyl-, Propyl-, Isopropyl-, Isobutyl-, sek.-Butyl-, tert.-Butyl-, Pentyl-, Isopentyl-, Hexyl-, Benzyl-, Phenylgruppen einfach oder gleich oder unterschiedlich zweifach substituierte 1,1,1-Trichloralkane.

Die Umsetzung wird im allgemeinen bei einer Temperatur von 30 bis 150°C, vorzugsweise von 50 bis 120°C, insbesondere von 80 bis 105°C, mit Unterdruck, Überdruck oder drucklos, vorzugsweise drucklos, diskontinuierlich oder kontinuierlich durchgeführt. Sie erfolgt in Gegenwart vorgenannter Mono-, Di- oder Polyole, Tri-, Di- oder Monoäthanolamine als Katalysatoren. Bevorzugte Katalysatoren sind solche, in deren Formeln $R^3$, $R^4$ und $R^5$ gleich oder verschieden sein können

und für ein Wasserstoffatom oder einen Alkylrest mit 1 bis 12, insbesondere 1 bis 6 Kohlenstoffatomen bezeichnen, n, m und o gleich oder verschieden sein können und jeweils eine Zahl von 1 bis 10, insbesondere 1 bis 4 bedeuten, m und o auch für 0 stehen können. Die Katalysatoren der Formeln III bis VIII werden in Mengen von 1 bis 50 Gew.%, bevorzugt 20 bis 40 Gew.%, bezogen auf die 1,1,1-Trichlorverbindung II, eingesetzt. Es können Katalysatorgemische verwendet werden.

Es kommen z.B. in Frage als Beispiele für Verbindungen der Formel III:

| | |
|---|---|
| $H(OCH_2CH_2)_nOH$ | n = 2, 3, 4, 5...10 |
| $CH_3(OCH_2CH_2)_nOH$ | n = 1, 2, 3, 4, 5...10 |
| $C_2H_5(OCH_2CH_2)_nOH$ | n = 1, 2, 3, 4, 5...10 |
| $n\text{-}C_4H_9(OCH_2CH_2)_nOH$ | n = 1, 2, 3, 4, 5...10 |
| $CH_3(OCH_2\text{-}\underset{CH_3}{CH})_nOH$ | n = 1, 2... 10 |
| $C_2H_5(OCH_2\underset{CH_3}{CH})_nOH$ | n = 1, 2... 10 |
| $n\text{-}C_4H_9(OCH_2\underset{CH_3}{CH})_nOH$ | n = 1, 2... 10 |

Beispiele für Verbindungen der Formel IV:

| | |
|---|---|
| $CH_2\text{-}(OCH_2CH_2)_nOH$ | n = 1, 2...10 |
| $CH\ \text{-}(OCH_2CH_2)_mOH$ | m = 0, 1, 2...10 |
| $CH_2\text{-}(OCH_2CH_2)_oOH$ | o = 0, 1, 2...10 |

Beispiele für Verbindungen der Formel V:

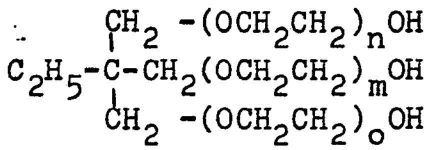

n = 1, 2...10
n = 0, 1, 2...10
o = 0, 1, 2...10

Beispiele für Verbindungen der Formel VII:

$CH_3N(CH_2CH_2OH)_2$
$C_2H_5N(CH_2CH_2OH)_2$
$i-C_3H_7N(CH_2CH_2OH)_2$
$n-C_4H_9N(CH_2CH_2OH)_2$

Beispiele für Verbindungen der Formel VIII:

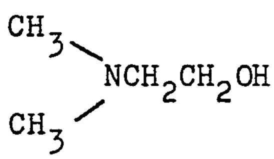

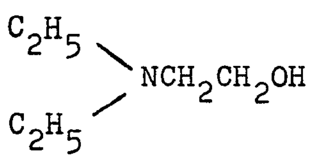

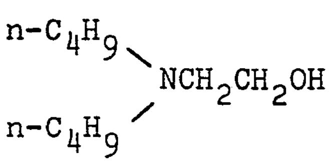

Die Reaktion kann wie folgt durchgeführt werden: Ein Gemisch von Ausgangsstoff II, Alkalihydroxid, Wasser, Katalysator wird während 0,5 bis 10 Stunden bei der Reaktionstemperatur gehalten. Dann wird aus dem Reaktionsgemisch der Endstoff in üblicher Weise, z.B. durch Waschen mit Wasser zur Entfernung des Alkalichlorids und Destillation abgetrennt. Alle Komponenten können vor der Reaktion gemischt und gemeinsam erhitzt werden. Bevorzugt erhitzt man jedoch

ein Gemisch aus dem Katalysator und der 1,1,1-Trichlorverbindung II auf die Reaktionstemperatur und läßt das in Wasser gelöste Alkalihydroxid in 0,5 bis 4 Std. zulaufen. Man kann auch zusammen mit dem Katalysator das Hydroxid vorlegen und die Trichlormethylverbindung II zulaufen lassen. Nach Beendigung des Zulaufs wird zweckmäßig noch 0,5 bis 10 Std. nacherhitzt.

Die unter den vorgenannten Bedingungen erhaltenen 1,1-Trichloralkenylenverbindungen sind Monomere und wertvolle Ausgangsstoffe für die Herstellung von Farbstoffen, Pharmazeutika und Pflanzenschutzmitteln. So können sie beispielsweise auch als Zwischenprodukte für PVC-Kunststoffe und Pyrethroide verwendet werden.

Die in den folgenden Beispielen aufgeführten Teile bedeuten Gewichtsteile. Sie verhalten sich zu den Volumenteilen wie Kilogramm zu Liter.

Beispiel 1

Ein Gemisch aus 225 Teilen 1,1,1-Trichloräthan, 70 Teilen Triglykolmono-n-butylether und 210 Teilen 50 gew.%iger wäßriger Natronlauge wird unter Rühren während 30 Minuten auf 70°C bis 80°C erhitzt. Über eine mit 5 mm-Glasringen gefüllte 12 cm hohe Kolonne destilliert man in 4 Stunden 144 Teile 1,1-Dichloräthylen (88 % der Theorie) bei der Übergangstemperatur 32°C ab.

Beispiel 2

450 Teile 1,1,1-Trichlor-4-methylpenten-3 und 140 Teile Triglykolmonobutylether werden auf 105°C geheizt. Unter Rühren läßt man in 2 Stunden 200 Vol.Teile 50 gew.%ige Natronlauge zulaufen und heizt anschließend noch 2 Stunden nach.

Nach dem Abkühlen wird das bei der Reaktion ausgefallene Kochsalz durch Zusatz von 490 Teilen Wasser in Lösung gebracht. Nach Trennung der Phasen destilliert man das 1,1-Dichlor-4-methylpentadien-1,3 bei 117°C/200 mbar. Man erhält 319 Teile (88 % der Theorie).

Beispiel 3

Die Umsetzung von Beispiel 2 wird mit Triglykolmonomethylether wiederholt. Man erhält 326 Teile (90 % der Theorie) 1,1-Dichlor-4-methylpentadien-1,3 von 117°C/200 mbar.

Beispiel 4

Die Umsetzung von Beispiel 2 wird mit Diglykolmonobutylether wiederholt. Man erhält 316 Teile (87 % der Theorie) 1,1-Dichlor-4-methylpentadien-1,3 vom Kp. 117°C (200 mbar).

Beispiel 5

600 Teile 1,1,1-Trichlor-4-methylpentanol-4 und 200 Teile Triglykolmonobutylether werden auf 110°C erhitzt. In 90 Minuten läßt man 260 Vol.Teile 50 gew.%ige wäßrige Natronlauge zulaufen und kocht anschließend noch 6 Stunden unter Rückfluß. Nach dem Abkühlen werden 700 Teile Wasser zugesetzt. Durch Destillation der organischen Phase erhält man 385 Teile (78 % der Theorie) 1,1-Dichlor-4-methylpenten-1-ol-4. Kp 80°C/16 mbar.

Vergleichsbeispiel 6

26,7 Teile 1,1,1-Trichlorethan und 48 Gew.-Teile 50%ige Natronlauge werden 7 Stunden lang unter Rühren gekocht. Eine anschließende gaschromatographische Analyse zeigt, daß sich

neben unverändertem Ausgangsstoff 0,1 % 1,1-Dichlorethylen gebildet haben.

Vergleichsbeispiel 7

45 Teile 1,1,1-Trichlor-4-methylpenten-3 und 20 Vol.-Teile 50 gew.%ige Natronlauge werden 10,5 Stunden bei 105°C gehalten. Eine anschließende gaschromatographische Analyse zeigt, daß der Ausgangsstoff noch unverändert vorliegt.

## Patentanspruch

Verfahren zur Herstellung von 1,1-Dichloralkenylen-Verbindungen der Formel I

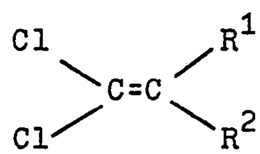

I,

worin $R^1$ und $R^2$ gleich oder verschieden sein können und jeweils ein Wasserstoffatom oder einen aliphatischen Rest bedeuten, $R^1$ auch einen araliphatischen oder aromatischen Rest bezeichnet, durch Umsetzung von 1,1,1-Trichlorverbindungen der Formel II

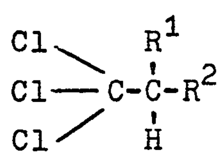

II,

worin $R^1$ und $R^2$ die vorgenannte Bedeutung haben, mit wäßrigen Alkalilaugen, <u>dadurch gekennzeichnet</u>, daß man die Umsetzung in Gegenwart eines Mono- oder Polyols der Formel

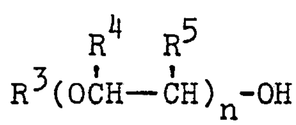

III,

in der $R^3$, $R^4$ und $R^5$ gleich oder verschieden sein können und für ein Wasserstoffatom oder einen aliphatischen Rest stehen und n eine ganze Zahl von 1 bis 10 bedeutet oder der Formel

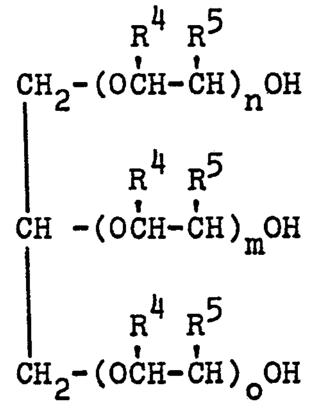 IV,

oder der Formel

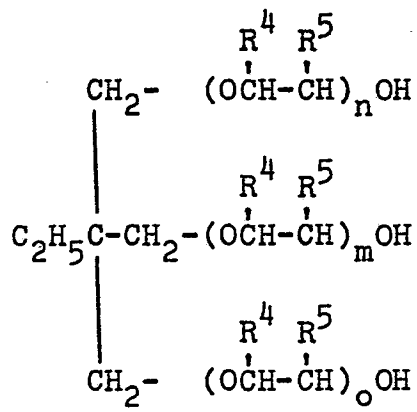 V,

wobei $R^4$, $R^5$ und n die oben angegebene Bedeutung haben und n, m, o gleich oder verschieden sein können und m und o eine ganze Zahl von 0 bis 10 bedeuten, oder der Formel

$$N(CH_2CH_2OH)_3 \qquad VI$$

oder der Formel

$$R^4N(CH_2CH_2OH)_2 \qquad VII,$$

in der $R^4$ die oben angegebene Bedeutung hat, oder der Formel

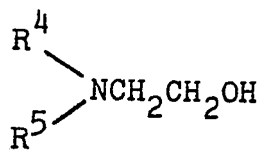

VIII,

in der $R^4$ und $R^5$ die oben angegebene Bedeutung haben, durchführt.

Europäisches Patentamt

# EUROPÄISCHER RECHERCHENBERICHT

0053687
Nummer der Anmeldung

EP 81 10 8692.5

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| Y | WO - A1 - 80/01910 (BP CHEMICALS)<br>* Ansprüche 1, 3 *<br>-- | 1 |
| Y | DE - A1 - 2 707 073 (BP CHEMICALS)<br>* Ansprüche 1, 7 *<br>-- | 1 |
| Y | DE - A1 - 2 503 826 (PETRO-TEX CHEMICAL)<br>* Ansprüche 1, 8; Tabellen 1, 2 *<br>-- | 1 |
| Y | CH - A - 231 886 (UNION DES FABRIQUES BELGES DE TEXTILES ARTIFICIELS FABELTA)<br>* Anspruch 1 *<br>-- | 1 |
| Y | GB - A - 1 173 359 (DU PONT DE NEMOURS)<br>* Ansprüche 1, 21 *<br>-- | 1 |
| Y | GB - A - 1 235 591 (DU PONT DE NEMOURS)<br>* Ansprüche 1, 28, 39 *<br>& DE - A - 1 909 952<br>---- | 1 |

**KLASSIFIKATION DER ANMELDUNG (Int. Cl.³)**

C 07 C 17/34
C 07 C 21/04
//C 07 C 21/073
C 07 C 21/19

**RECHERCHIERTE SACHGEBIETE (Int. Cl.³)**

C 07 C 17/34
C 07 C 21/02
C 07 C 21/04
C 07 C 21/073
C 07 C 21/19

**KATEGORIE DER GENANNTEN DOKUMENTE**

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze
E: älteres Patentdokument, das jedoch erst am oder nach dem Anmeldedatum veröffentlicht worden ist
D: in der Anmeldung angeführtes Dokument
L: aus andern Gründen angeführtes Dokument

&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Berlin | 25-02-1982 | KNAACK |

EPA form 1503.1 06.78